# EUROPEAN PATENT APPLICATION

(11) **EP 2 471 378 A1**
(43) Date of publication of application: **04.07.2012**
(21) Application number: 12162689.9
(22) Date of filing: 03.07.2007
(51) Int. Cl.: A23K 1/175, A23K 1/18, A61K 33/04, A61K 33/18, A61P 5/14

(54) **Compositions and methods for preventing or treating cardiovascular diseases in felines with hyperthyroidism**

(30) Priority: 03.07.2006 US 818428 P
(62) Divisional of application: 07799274.1
(71) Applicant: Hill's Pet Nutrition, Inc., Topeka, KS 66603 (US)
(72) Inventor: Wedekind, Karen, Joy, Meriden, KS Kansas 66512 (US); Kirk, Claudia, Ann, Knoxville, TN Tennessee 37919 (US)
(74) Representative: Daniels, Jeffrey Nicholas

(57) **Abstract**

The invention relates to compositions for preventing and/or treating cardiovascular diseases in felines with hyperthyroidism wherein the compositions comprise from about 0.07 to less than about 1 mg/kg iodine and/or from about 0.1 to about 1.3 mg/kg selenium. The invention also relates to methods for preparing and using such compositions, articles of manufacture comprising such compositions, and means for communicating information about such compositions, methods, and articles of manufacture.

## Description

### CROSS REFERENCE TO RELATED APPLICATION

This application clauses the benefit of U.S. Patent Application No. 60/818,428, filed July 03, 2006.

### FIELD OF THE INVENTION

This invention relates generally to compositions and methods for preventing or treating cardiovascular diseases and particularly to compositions and methods for preventing or treating cardiovascular diseases in felines with hyperthyroidism.

### BACKGROUND OF THE INVENTION

Hyperthyroidism is characterized by hypermetabolism of the thyroid gland and excessive production of the thyroid hormones triiodothyronine (T₃) and tetraiodothyronine (thyroxine or T₄). Felines with hyperthyroidism may exhibit one or more cardiovascular abnormalities. For example, on physical examination, felines with hyperthyroidism often exhibit systolic murmurs, tachycardia, and gallop rhythm. Various arrhythmias and signs of chronic heart failure (e.g., dyspnea, muffled heart sounds, ascites) may also be detected upon physical examination. Radiographic findings in felines with hyperthyroidism include mild to severe cardiomegaly, pleural effusion, and pulmonary edema. Electrocardiographic abnormalities in felines with hyperthyroidism include tachycardia, increased R-wave amplitude in lead II, various ventricular and atrial arrhythmias, and intraventricular conduction disturbances. Echocardiographic abnormalities in hyperthyroid felines include left ventricular hypertrophy, thickening of the interventricular septum, left ventricular and atrial dilation, myocardial hypercontractility, and dilative cardiomyopathy.

Although the exact pathogenesis of cardiac abnormalities associated with hyperthyroidism in unclear, it is believed that cardiac changes that compensate for altered peripheral function caused by hyperthyroidism play an important role. Hyperthyroidism results in a high-output cardiac state in which vascular resistance is low and cardiac output is high because of increased tissue metabolism and oxygen requirements. A volume overload is created by low peripheral vascular resistance and by reflex renal mechanisms that conserve fluid. The principal cardiac compensatory mechanisms in hyperthyroidism are dilation (in response to volume overload) and hypertrophy (in response to dilation). The direct action of thyroid hormones on heart muscle and the interactions of T₃ and T₄ with the sympathetic nervous system, also appear to influence cardiac function in byperthyroidism.

Hyperthyroidism can induce a hypertrophic form of cardiomyopathy and, less commonly, a dilative type of cardiomyopathy. Hypertrophic cardiomyopathy is a heart muscle disease in which the muscular walls of the left ventricle become abnormally thickened. Internal cardiac structures, such as the papillary muscles, also thicken. Either form of cardiomyopathy may result in chronic heart failure because presence of excess thyroid hormone may cause irreversible cardiac structural damage. In addition, hyperthyroidism may aggravate a pre-existing cardiovascular disease.

Thus, there is a need for compositions and methods for treating cardiovascular diseases in felines with hyperthyroidism that provide partial or complete relief. In addition, there is a need for compositions and methods for preventing cardiovascular diseases in felines with hyperthyroidism.

### SUMMARY OF THE INVENTION

It is an object of the invention to provide compositions suitable for preventing cardiovascular disease in felines with hyperthyroidism.

It is another object of the invention to provide compositions suitable for treating cardiovascular disease in felines with hyperthyroidism.

It is another object of the invention to provide methods for preventing cardiovascular diseases in felines with hyperthyroidism.

It is another object of the invention to provide methods for treating cardiovascular disease in felines with hyperthyroidism.

It is another object of the invention to provide articles of manufacture comprising a composition of the invention or two or more ingredients that, when combined together and, optionally, with additional ingredients, yield a composition for preventing cardiovascular disease in a feline with hyperthyroidism.

It is another object of the invention to provide articles of manufacture comprising a composition of the invention or two or more ingredients that, when combined together and, optionally, with additional ingredients, yield a composition for treating cardiovascular disease in a feline with hyperthyroidism.

It is another object to provide means for communicating information about the compositions, methods, and articles of manufacture of the invention.

Additional or alternative advantages and benefits of the present invention will be apparent to one of skill in the art.

### DETAILED DESCRIPTION OF THE INVENTION

Hyperthyroidism is characterized by hypermetabolism of the thyroid gland and the excessive production of the thyroid hormones T₃ and T₄. Most of T₃ and T₄ are bound to serum proteins. One skilled in the art can accurately diagnose hyperthyroidism in a feline utilizing thyroid function tests, examining clinical signs, and/or observing the animal's response to trial thyroid hormone administration. Thyroid function tests are known to those skilled in the art and include, for example, tests for determining the concentrations of total and free serum T₃ and T₄, tests for determining the concentration of thyroid stimulating hormone, and the sodium pertechnetate and T₃ suppression tests. See, for example, Small Animal Nutrition, pages 863-868 (2000).

As discussed above, hyperthyroidism is a factor in developing a variety of cardiovascular abnormalities, which, in turn, may negatively affect a feline's lifestyle and may even lead to death. It is, therefore, important to prevent cardiovascular diseases in felines that have been diagnosed with hyperthyroidism, but do not present with clinical signs of such diseases. Thus, in one aspect, the invention provides a composition for preventing one or more cardiovascular diseases in a feline with hyperthyroidism that may be susceptible to develop cardiovascular disease. Such a feline is one that has not been diagnosed with cardiovascular disease, but is at risk to develop cardiovascular disease. Preventing cardiovascular disease as used herein includes reducing the risk of, delaying the onset of, and/or keeping a hyperthyroid feline from developing a cardiovascular disease. Thus, feeding a composition for preventing cardiovascular disease can reduce a feline's risk of developing cardiovascular disease(s). In addition, feeding such a composition can slow down the progression of hyperthyroidism, thereby delaying the onset of cardiovascular disease.

Treatment can help a hyperthyroid feline with cardiovascular disease to live a healthier, longer, and more active life. Thus, in another aspect, the present invention provides a composition for treating one or more cardiovascular diseases and/or hyperthyroidism in a hyperthyroid feline in need of such treatment. Treating cardiovascular disease includes ameliorating, suppressing, and/or delaying cardiovascular disease. One skilled in the art can diagnose a variety of cardiovascular diseases by utilizing, for example, physical examination, radiography, electrocardiography, and/or echocardiography.

The compositions for preventing and treating of cardiovascular disease(s) are suitable for any feline with hyperthyroidism that is susceptible to develop cardiovascular diseases. In some embodiments, the feline is a companion feline. A companion feline can be a feline kept as a pet. A companion feline can also be a feline from a widely domesticated species, for example, cats *(Felis domesticus)* regardless of whether or not it is kept as a pet. In some embodiments, the feline is an adult feline. An adult feline is a feline of any age after juvenile growth and development has been completed, including senior and geriatric felines. For example, an adult cat typically is one that is from about one year old through the remainder of its life. A senior feline is one of an age at which it is at a risk for suffering from an age-related disease regardless of whether or not the feline shows obvious physical or behavioral signs of aging. For example, a senior cat typically is a cat from about seven to about eleven years old. A geriatric feline is a feline showing signs of aging. For example, a geriatric cat typically is a cat of about twelve years of age and beyond.

The compositions for preventing and treating of cardiovascular disease(s) are suitable for any cardiovascular disease, for example, cardiac hypertrophy, systolic murmurs, gallop rhythm, chronic heart failure, cardiomegaly, pleural effusion, pulmonary edema, increased R-wave amplitude in lead II, tachycardia, ventricular arrhythmia, intraventricular conduction disturbance, left ventricular hypertrophy, thickening of the interventricular septum, left ventricular dilation, left atrial dilation, myocardial hypercontractility, dilative cardiomyopathy, hypertrophic cardiomyopathy, and atrial arrhythmia. The compositions of the invention for treating a cardiovascular disease can be used to treat cardiovascular diseases secondary to hyperthyroidism (i.e., cardiovascular conditions that are the result of developing hyperthyroidism) as well as primary cardiovascular diseases (i.e., cardiovascular diseases that the feline suffered from before developing hyperthyroidism). In some embodiments, the cardiovascular disease comprises cardiac hypertrophy. In some embodiments, the cardiovascular disease comprises hypertrophic cardiomyopathy.

As used herein, the term "about" means the variance in the numbers for mg/kg are plus or minus 0.05 mg/kg; and in the percentages are plus or minus 0.5%. For example, "about 1.7 mg/kg" means 1.65 to 1.75 mg/kg; and "about 10%" means 9.5 to 10.5%.

In an embodiment, the composition for preventing and/or treating a cardiovascular disease in a feline with hyperthyroidism comprises from about 0.07 to less than about 1 mg/kg iodine. In some such embodiments, the composition comprises from about 0.07 to about 0.5 mg/kg iodine. In other such embodiments, the composition comprises from about 0.07 to about 0.3 mg/kg iodine. In yet other such embodiments, the composition comprises from about 0.15 to about 0.25 mg/kg iodine. And in further such embodiments, the composition comprises from about 0.07 to about 0.2 mg/kg iodine.

In some embodiments, the composition for preventing and/or treating a cardiovascular disease in a feline with hyperthyroidism comprises from about 0.1 to less than about 1 mg/kg iodine. In some such embodiments, the composition comprises from about 0.1 to about 0.5 mg/kg iodine. In other such embodiments, the composition comprises from about 0.1 to about 0.3 mg/kg iodine. In yet other such embodiments, the composition comprises from about 0.15 to about 0.25 mg/kg iodine. And in further such embodiments, the composition comprises from about 0.1 to about 0.2 mg/kg iodine. Iodine is a constituent of T₃ and T₄. The thyroid glands actively trap iodine to ensure an adequate supply of thyroid hormones. Iodine as used herein refers to the iodine atom without reference to its molecular or ionic form, and includes iodine present in one or more chemical forms such as, for example, iodide, iodate, and periodate.

In some embodiments, the composition of the invention for preventing and/or treating a cardiovascular disease in a feline with hyperthyroidism comprises from about 0.1 to about 1.3 mg/kg selenium. In other such embodiments, the composition of the invention for preventing and/or treating a cardiovascular disease in a feline with hyperthyroidism comprises from about 0.1 to about 1 mg/kg selenium. In some such embodiments, the composition comprises from about 0.1 to about 0.8 mg/kg selenium. In other such embodiments, the composition comprises from about 0.15 to about 0.65 mg/kg selenium. In yet other such embodiments, the composition comprises from about 0.4 to about 0.7 mg/kg selenium. In further such embodiments, the composition comprises from about 0.3 to about 0.65 mg/kg selenium. Selenium has a role in maintaining normal thyroid and iodine metabolism, particularly through the control of the deiodinase enzymes that regulate the conversion of T₄ to T₃. Selenium as used herein refers to the selenium atom without reference to its molecular or ionic form, and includes selenium present in one or more chemical forms such as, for example, selenide, selenite, and selenate.

In some embodiments, the composition of the invention for preventing and/or treating a cardiovascular disease in a feline with hyperthyroidism comprises from about 0.07 to less than about 1 mg/kg iodine as well as from about 0.1 to less than about 1.3 mg/kg selenium. In some embodiments, the composition comprises from about 0.07 to less than about 1 mg/kg iodine as well as from about 0.1 to less than about 1 mg/kg selenium. In some such embodiments, the composition comprises from about 0.07 to about 0.5 mg/kg iodine and from about 0.1 to about 0.8 mg/kg selenium. In other such embodiments, the composition comprises from about 0.07 to about 0.3 mg/kg iodine and from about 0.15 to about 0.65 mg/kg selenium. In further such embodiments, the composition comprises (1) from about 0.07, about 0.1, about 0.15, or about 0.2 to about 0.25, about 0.3, or about 0.5 mg/kg iodine, and (2) from about 0.1, about 0.15, about 0.3, or about 0.4 to about 0.65, about 0.7, or about 0.8 mg/kg selenium.

In some embodiments, the composition of the invention for preventing and/or treating a cardiovascular disease in a feline with hyperthyroidism comprises from about 0.1 to less than about 1 mg/kg iodine as well as from about 0.1 to less than about 1 mg/kg selenium. In some such embodiments, the composition comprises from about 0.1 to about 0.5 mg/kg iodine and from about 0.1 to about 0.8 mg/kg selenium. In other such embodiments, the composition comprises from about 0.1 to about 0.3 mg/kg iodine and from about 0.15 to about 0.65 mg/kg selenium. In further such embodiments, the composition comprises (1) from about 0.1, about 0.15, or about 0.2 to about 0.25, about 0.3, or about 0.5 mg/kg iodine, and (2) from about 0.1, about 0.15, about 0.3, or about 0.4 to about 0.65, about 0.7, or about 0.8 mg/kg selenium.

Tables 2 and 3 of U.S. Patent Application No. US 2005/0058691 Al list the iodine and selenium content of commercially available canned and dry cat foods. The average amounts of selenium in the 28 tested canned foods and 14 tested dry foods are 1.77 and 0.69 mg/kg, respectively, with many foods having more than 2 mg/kg. The average amounts of iodine in those foods are 7.83 mg/kg and 2.77 mg/kg, respectively, with some foods having more than 30 mg/kg. Thus, the compositions comprise amounts of iodine and/or selenium that are lower (and in some embodiments, much lower) than the amounts of iodine and selenium in many commercially available foods. Thus, in some embodiments, feeding a composition to a feline results in restricting the feline's intake of iodine. In other embodiments, feeding a composition to a feline results in restricting the feline's intake of selenium. In further embodiments, feeding a composition to a feline results in restricting the feline's intake of both iodine and selenium.

In some embodiments, the composition of the invention for preventing and/or treating a cardiovascular disease in a feline with hyperthyroidism comprises from about 31 to about 35% protein. The protein present in the composition comprises at least about 75% vegetable protein. Commercial adult cat foods, for example, typically comprise from about 35 to about 45% protein. Thus, in many embodiments, a composition for preventing and/or treating a cardiovascular disease in a hyperthyroid feline comprises less protein than most commercially available adult cat foods. In some such embodiments, the composition of the invention comprises from about 32 to about 34% protein. In other embodiments, the composition comprises from about 32 to about 35% protein. In other embodiments, the composition comprises from about 33 to about 35% protein. In other embodiments, the composition comprises from about 31, about 32, or about 33 to about 34 or about 35% protein. In further embodiments, the composition comprises from about 31 to about 32, about 33, about 34, or about 35% protein.

Because felines are carnivores, feline foods typically are formulated to comprise mostly animal and fish protein. To the contrary, the protein in a composition of the present invention for preventing and/or treating a cardiovascular disease in a feline with hyperthyroidism comprises at least about 75% vegetable protein. In some such embodiments, the composition comprises at least about 80% vegetable protein. In other embodiments, the composition comprises at least about 85% vegetable protein. In other such embodiments, the protein comprises at least about 90% vegetable protein. And in other such embodiments, the protein comprises at least about 95% vegetable protein (i.e., from at least about 95 up to about 100% vegetable protein). Thus, in other embodiments, the composition for preventing and/or treating a cardiovascular disease in a feline with hyperthyroidism comprises from about 31 to about 35% protein wherein the protein comprises at least about 75% vegetable protein. In some such embodiments, the composition comprises from about 31, about 32, or about 33 to about 34 or about 35% protein wherein the protein comprises at least about 75, at least about 80, at least about 85, at least about 90, or at least about 95% vegetable protein. In other embodiments, the composition comprises from about 31 to about 32, about 33, about 34, or about 35% protein, and the protein comprises at least about 75, at least about 80, at least about 85, at least about 90, or at least about 95% vegetable protein. In yet other embodiments, the composition comprises from about 28 or about 28.5 to about 29, about 29.5, or about 30% protein, and the protein comprises at least about 75, at least about 80, at least about 85, at least about 90, or at least about 95% vegetable protein.

One skilled in the art would understand that a composition for preventing and/or treating a cardiovascular disease in a feline with hyperthyroidism can comprise any of the combinations of iodine, selenium, and protein (including varying amounts of vegetable protein) discussed above. Thus, for example, in some embodiments, the composition for preventing and/or treating a cardiovascular disease in a feline with hyperthyroidism comprises from about 0.07, about 0.1, about 0.15, or about 0.2 to about 0.25, about 0.3, or about 0.5 mg/kg iodine, and/or from about 0.1, about 0.15, about 0.3, or about 0.4 to about 0.65, about 0.7, or about 0.8 mg/kg selenium, and, optionally from about 31, about 32, or about 33 to about 34 or about 35% protein wherein the protein comprises at least about 75, at least about 80, at least about 85, at least about 90, or at least about 95% vegetable protein.

In some embodiments, the composition for preventing and/or treating a cardiovascular disease in a hyperthyroid feline as described above further comprises one or more antithyroid agents and/or one or more anti-cardiovascular disease agents.

An antithyroid agent is a compound, a derivative thereof (e.g., a salt, solvate, or hydrate of the compound), or a composition comprising such compounds and/or derivatives that is used to treat hyperthyroidism. Suitable antithyroid agents include, for example, thioureylenes (e.g., methimazole, propylthiouracil, and carbimazole), aniline derivatives (e.g., sulfonamides), polyhydric phenols (e.g., resorcinol), and lithium salts. In some embodiments, the antithyroid agent comprises a thioureylene. Thioureylenes are five- or six-member thiourea derivatives that block thyroid hormone production. In some such embodiments, the antithyroid agent comprises the thioureylene methimazole. In other such embodiments, the antithyroid agent comprises the thioureylene propylthiouracil. In further such embodiments, the antithyroid agent comprises the thioureylene carbimazole. An anti-cardiovascular disease agent is a compound, derivative thereof (e.g., a salt, solvate, or hydrate of the compound), or a composition comprising such compounds and/or derivatives used to treat a cardiovascular disease. Suitable anti-cardiovascular disease agents include, for example, diuretics, angiotensin converting enzyme inhibitors, angiotensin H receptor antagonists, vasodilators, alpha adrenergic receptor antagonists, endothelin receptor antagonists, calcium channel blockers, and beta adrenergic receptor antagonists.

In some embodiments, the composition of the invention for preventing and/or treating a cardiovascular disease comprises one or more antithyroid agents, and the total amount of the antithyroid agents is a therapeutically-effective amount ("a therapeutically-effective amount of an antithyroid agent"). A therapeutically-effective or effective amount is an amount that will achieve the goal of treating the targeted condition. Those skilled in the art either know or can determine by routine experimentation how much of an agent or combination of agents to administer to a feline to treat a specific condition (e.g., hyperthyroidism). For example, one skilled in the art can prepare a composition that, when fed to the feline in a maintenance-sufficient amount, typically will deliver a therapeutically-effective amount of the agent(s) present in the composition. In some cases, the amount of the agent may vary with the stage of treatment. For example, higher doses of an antithyroid agent may be used in the initial stage of treatment. Thus, the therapeutically-effective amount of the particular agent may vary with the stage of the disease. One skilled in the art can prepare compositions with varying amounts of an antithyroid agent, and then feed those compositions sequentially to deliver the desired amount of antithyroid agent(s) to the feline. In some embodiments, the composition comprises one or more anti-cardiovascular disease agents, and the total amount of the anti-cardiovascular disease agents is a therapeutically-effective amount ("a therapeutically-effective amount of an anti-cardiovascular agent"). In some embodiments, the composition comprises a therapeutically-effective amount of an antithyroid agent and a therapeutically-effective amount of an anti-cardiovascular disease agent.

In some embodiments, the compositions for preventing and/or treating a cardiovascular disease in a hyperthyroid feline comprise one or more food compositions. In some embodiments, the compositions meet AAFCO's minimum nutrient level requirements for maintenance. While foods of any consistency or moisture content are contemplated, the compositions of the present invention may be, for example, a wet, dry, or semi-moist animal food composition. "Wet" food refers to food that has a moisture content of about 70 to about 90%. "Dry" food refers to compositions with about 5 to about 15% moisture content and is often manufactured in the form of small bits or kibbles. "Semi-moist" food refers to compositions with about 25 to about 45% moisture content. Also contemplated herein are compositions that may comprise components of various consistency as well as components that may include more than one consistency, for example, soft, chewy meat-like particles as well as kibble having an outer cereal component and an inner cream component as described in, e.g., U.S. Patent No. 6,517,877. In some embodiments, the food compositions comprise a treat, snack, supplement, or partially or fully edible toy. In some embodiments, the compositions comprise a mixture of one or more foods.

In another aspect, the invention provides methods for preparing compositions for preventing and/or treating a cardiovascular disease in a feline with hyperthyroidism. Such composition can be prepared, for example, by mixing various ingredients (including food compositions) that, when combined, yield a composition of the invention. Compositions can also be prepared by the methods discussed in, for example, Small Animal Nutrition, pages 127-146 (2000). Plant ingredients suitable for preparing compositions of the invention include, for example, potato concentrate, soy concentrate, soybean meal, soy protein isolate, corn gluten meal, rice protein isolate, pea protein concentrate, wheat protein concentrate, and wheat protein isolate. To prepare a low selenium-comprising composition, one can use, for example, a selenium-free mineral mix and ingredients that contain small amounts of selenium such as, for example, potato concentrate, soy concentrate, and soy protein isolate. To prepare a low iodine-comprising composition, one can, for example, avoid food colorings rich in iodine and can use an iodine-free mineral mix, non-iodized salt, or ingredients that contain small amounts of iodine such as, for example, potato concentrate, soy concentrate, and soy protein isolate.

Iodine and selenium-containing ingredients suitable for preparing a composition of the invention are listed in, for example, table 3 of U.S. Patent Application No. US 2005/0058691 Al. Plant ingredients suitable for preparing a composition include, for example, soybean meal, corn gluten meal, rice protein isolate, pea protein concentrate, wheat protein concentrate, and wheat protein isolate. Eggs can be used for preparing a composition as well. Meat ingredients suitable for preparing a composition include, for example, pork liver, beef spleen, beef tongue, pork lung lobes, beef lung, meat protein isolate, deboned turkey, chicken backs, and poultry by-product meal. One skilled in the art would understand that in some embodiments of the invention, one would need to utilize mostly plant ingredients to achieve high vegetable protein content as well as low iodine and/or selenium content. As discussed above, commercially available cat foods typically contain higher amounts of iodine and selenium than the amounts of iodine and/or selenium in a composition of the present invention.

In another aspect, the invention provides a method for preventing one or more cardiovascular diseases in a feline, susceptible to develop such a disease, with hyperthyroidism.

In another aspect, the invention provides a method for treating one or more cardiovascular diseases in a hyperthyroid feline in need of such treatment.

The methods of the invention for preventing and/or treating a cardiovascular disease in a feline with hyperthyroidism comprise feeding the feline a composition discussed above. One skilled in the art would understand that either a single composition can be fed to the feline or, alternatively, different such compositions can be fed to the feline for varying time intervals.

In some embodiments, the methods for preventing and/or treating a cardiovascular disease in a feline with hyperthyroidism comprise feeding the feline a composition that comprises from about 0.07 to less than about 1 mg/kg iodine. In some such embodiments, the composition fed to the feline comprises from about 0.1 to about 1 mg/kg iodine. In some such embodiments, the composition fed to the feline comprises from about 0.1 to about 0.5 mg/kg iodine. In other such embodiments, the composition fed to the feline comprises from about 0.1 to about 0.3 mg/kg iodine. In other such embodiments, the composition fed to the feline comprises from about 0.15 to about 0.25 mg/kg iodine.

In some embodiments, the methods for preventing and/or treating a cardiovascular disease in a feline with hyperthyroidism comprise feeding the feline a composition that comprises from about 0.1 to about 1.3 mg/kg selenium. In some such embodiments, the composition fed to the feline comprises from about 0.1 to about 0.8 mg/kg selenium. In other such embodiments, the composition fed to the feline comprises from about 0.15 to about 0.65 mg/kg selenium. In other such embodiments, the composition comprises from about 0.4 to about 0.7 mg/kg selenium. And in other such embodiments, the composition fed to the feline comprises from about 0.3 to about 0.65 mg/kg selenium.

In some embodiments, the methods for preventing and/or treating a cardiovascular disease in a feline with hyperthyroidism comprise feeding the feline a composition that comprises from about 0.07 to less than about 1 mg/kg iodine as well as from about 0.1 to less than about 1.3 mg/kg selenium. In some such embodiments, the composition fed to the feline comprises from about 0.1 to about 1 mg/kg iodine and from about 0.1 to about 0.8 mg/kg selenium. In some such embodiments, the composition fed to the feline comprises from about 0.1 to about 0.5 mg/kg iodine and from about 0.1 to about 0.8 mg/kg selenium. In other such embodiments, the composition fed to the feline comprises from about 0.1 to about 0.3 mg/kg iodine and from about 0.15 to about 0.65 mg/kg selenium. And in other such embodiments, the composition fed to the feline comprises (1) from about 0.07, about 0.1, about 0.15, or about 0.2 to about 0.25, about 0.3, or about 0.5 mg/kg iodine, and (2) from about 0. 1, about 0. 15, about 0.3, or about 0.4 to about 0.65, about 0.7, or about 0.8 mg/kg selenium.

In some embodiments, the methods for preventing and/or treating a cardiovascular disease in a feline with hyperthyroidism comprise feeding the feline a composition that comprises from about 0.07, about 0.1, about 0.15, or about 0.2 to about 0.25, about 0.3, or about 0.5 mg/kg iodine, and/or from about 0.1, about 0.15, about 0.3, or about 0.4 to about 0.65, about 0.7, or about 0.8 mg/kg selenium, and, optionally from about 31, about 32, or about 33 to about 34 or about 35% protein wherein the protein comprises at least about 75, at least about 80, at least about 85, at least about 90, or at least about 95% vegetable protein.

In some embodiments, the methods for preventing and/or treating a cardiovascular disease in a feline with hyperthyroidism further comprise administering to the feline one or more anti-cardiovascular disease agent. In some embodiments, the methods for prevention and treatment of a cardiovascular disease in a feline with hyperthyroidism further comprise administering to the feline one or more antithyroid agent. In some embodiments, the methods for prevention and treatment of a cardiovascular disease in a feline with hyperthyroidism further comprise administering to the feline one or more anti-cardiovascular disease agents and one or more antithyroid agents. Such agents are administered in conjunction with feeding a composition of the invention. The term "in conjunction" means that an agent is administered to the feline either together with a composition of the invention or separately from the composition at the same or different frequency via the same or different administration route and either at about the same time as the composition or periodically. "Administering" means that the agent is introduced in a suitable dosage form into the feline by a suitable administration route, for example, orally, topically, or parenterally. "About at the same time" generally means that an agent is administered when a composition is fed to the feline or within about 72 hours of feeding the composition to the feline. "Periodically" generally means that an agent is administered to a feline following a dosage schedule suitable for administering the agent while a composition is fed to the feline routinely as appropriate for that feline. Thus, the term "in conjunction" specifically includes situations when an agent is administered to a feline for a prescribed period of time while a composition is fed to the feline for a much longer period of time (e.g., for life). If more than one agent is administered, the dosage form and route of administration for each agent may vary. In addition, one composition may be substituted with another composition while a specific agent is administered to the feline.

Suitable anti-cardiovascular disease and antithyroid agents are discussed above in the context of the compositions of the invention. Such agents can be administered, for example, in the form of salts derived from inorganic or organic acids. Depending on the particular compound, a salt of the compound may be advantageous due to one or more of the salt's physical properties, for example, enhanced pharmaceutical stability in differing temperatures and humidities, or a desirable solubility in water or oil. The salt preferably is a pharmaceutically-acceptable salt.

The total daily dose of an agent (administered in single or divided doses) is typically from about 0.001 to about 100 mg/kg, preferably from about 0.001 to about 30 mg/kg, and even more preferably from about 0.01 to about 10 mg/kg body weight. Dosage unit compositions can contain such amounts or submultiples thereof to make up the daily dose. In many instances, the administration of an agent will be repeated a plurality of times. Multiple doses per day typically may be used to increase the total daily dose. Factors affecting the preferred dosage regimen include, for example, the age, weight, and condition of the feline; the severity of the disease; the route of administration; pharmacological considerations (e.g., activity, efficacy, and pharmacokinetic and toxicology profiles of the particular agent); whether a drug delivery system is utilized; and whether the agent is administered as part of a drug combination. Thus, the dosage regimen actually employed can vary widely, and can differ from the dosage regimen set forth above.

In another aspect, the invention provides a use of a composition of the invention to prepare a medicament to prevent a cardiovascular disease in a feline with hyperthyroidism.

In another aspect, the invention provides a use of a composition of the invention to prepare a medicament to treat a cardiovascular disease in a feline with hyperthyroidism.

In a further aspect, the invention provides an article of manufacture, for example, a kit comprising a composition of the invention as discussed above. The kit is suitable for preventing and/or treating a cardiovascular disease in a feline with hyperthyroidism. In some embodiments, the kit further comprises one or more anti-cardiovascular disease agents. In some embodiments, the kit further comprises one or more antithyroid agents. In some embodiments, the kit further comprises an anti-cardiovascular disease agent and an antithyroid agent. In some embodiments, the kit further comprises instructions for, for example, one or more of (a) feeding the composition to a feline with hyperthyroidism, (b) administering an anti-cardiovascular disease agent and/or an antithyroid agent to the feline in conjunction with feeding the feline the composition, (c) preventing cardiovascular disease in a feline with hyperthyroidism by feeding the feline the composition, (d) treating cardiovascular disease in a feline with hyperthyroidism by feeding the feline the composition, (e) preventing a cardiovascular disease in a feline with hyperthyroidism by administering to the feline an anti-cardiovascular disease agent and/or an antithyroid agent in conjunction with feeding the feline the composition, and (f) treating a cardiovascular disease in a feline with hyperthyroidism by administering to the feline an anti-cardiovascular disease agent and/or an antithyroid agent in conjunction with feeding the feline the composition.

In some embodiments of the invention, the kit comprises two or more ingredients that, when combined together and optionally with additional ingredients that are or are not a part of the kit, yield a composition of the present invention. If such additional ingredients are to be used, the kit provides instructions about these ingredients. In some embodiments, the kit further comprises an anti-cardiovascular disease agent. In some embodiments, the kit further comprises an antithyroid agent. In some embodiments, the kit further comprises an anti-cardiovascular disease agent and an antithyroid agent. In some embodiments, the kit further comprises instructions for one or more of (a) preparing a composition of the invention for preventing a cardiovascular disease in a hyperthyroid feline by combining the ingredients, (b) preparing a composition for treating a cardiovascular disease in a hyperthyroid feline by combining the ingredients, (c) feeding a feline with hyperthyroidism a composition for preventing a cardiovascular disease, (d) feeding a feline with hyperthyroidism a composition for treating a cardiovascular disease, (e) administering an anti-cardiovascular disease agent and/or an antithyroid agent to a feline in conjunction with feeding the feline a composition for preventing cardiovascular disease, (f) administering an anti-cardiovascular disease agent and/or an antithyroid agent to a feline with hyperthyroidism in conjunction with feeding the feline a composition for treating a cardiovascular disease, (g) preventing a cardiovascular disease in a hyperthyroid feline by feeding the feline a composition for preventing a cardiovascular disease, (h) treating cardiovascular disease in a feline with hyperthyroidism by feeding the feline a composition for treating cardiovascular disease, (i) preventing cardiovascular disease in a feline with hyperthyroidism by administering to the feline an anti-cardiovascular disease agent and/or an antithyroid agent in conjunction with feeding the feline a composition for preventing a cardiovascular disease, and j) treating a cardiovascular disease in a feline with hyperthyroidism by administering to the feline an anti-cardiovascular disease agent and/or an antithyroid agent in conjunction with feeding the feline a composition for treating a cardiovascular disease.

In some embodiments, the kit comprises in separate containers in a single package or in separate containers in a virtual package, as appropriate, a composition or two or more ingredients, that, when combined together and optionally with additional ingredients that are or are not a part of the kit, yield a composition of the invention, and instructions for one or more of (a) preparing a composition of the invention for preventing and/or treating a cardiovascular disease in a feline with hyperthyroidism by combining the ingredients, (b) feeding a hyperthyroid feline with a composition to prevent and/or treat cardiovascular disease, (c) administering an anti-cardiovascular disease agent and/or an antithyroid agent to a feline with hyperthyroidism in conjunction with feeding the feline a composition of the invention, (d) preventing and/or treating a cardiovascular disease in a feline with hyperthyroidism by feeding the feline a composition of the invention, (e) preventing and/or treating cardiovascular disease in a feline with hyperthyroidism by administering to the feline an anti-cardiovascular disease agent and/or an antithyroid agent in conjunction with feeding the feline a composition of the invention.

The term "single package" generally means that the components of a kit are physically associated in or with one or more containers and considered as a unit of manufacture, distribution, sale, or use. Containers include, for example, bags, boxes, bottles, shrink wrap packages, stapled or otherwise fixed components, and combinations thereof. A single package can be, for example, containers or individual food compositions physically associated such that they are considered a unit for manufacture, distribution, sale, or use. The term "virtual package" generally means that the components of a kit are associated by directions on one or more physical or virtual kit components instructing the user how to obtain additional components, e.g., in a bag containing one component and directions instructing the user to go to a website, contact a recorded message, view a visual message, or contact a caregiver to obtain instructions on how to use the kit. When the kit comprises a virtual package, the kit is limited to instructions in a virtual environment with one or more physical kit components.

In some embodiments, the kit is suitable for preventing and/or treating cardiovascular disease in a feline with hyperthyroidism and comprises in separate containers in a single package or in separate containers in a virtual package, as appropriate, a composition that comprises from about 0.07 to less than about 1 mg/kg iodine and/or from about 0.1 to about 1.3 mg/kg selenium, or two or more ingredients that, when combined together and, optionally, with additional ingredients that are or are not a part of the kit, yield a composition that comprises from about 0.07 to less than about 1 mg/kg iodine and/or from about 0.1 to about 1.3 mg/kg selenium, and one or more of (a) an anti-cardiovascular disease agent, (b) antithyroid agent, (c) instructions for preparing the composition, (d) instructions for feeding the composition to a feline with hyperthyroidism, (e) instructions for preventing and/or treating cardiovascular disease in a hyperthyroid feline with by feeding the feline the composition, and (f) instructions for preventing and/or treating a cardiovascular disease in a hyperthyroid feline by administering to the feline an anti-cardiovascular disease agent and/or an antithyroid agent in conjunction with feeding the feline the composition.

In a further aspect, the invention provides a means for communicating information about or instructions for one or more of (a) feeding a feline with hyperthyroidism composition to prevent cardiovascular disease, (b) feeding a feline with hyperthyroidism a composition to treat a cardiovascular disease, (c) administering an anti-cardiovascular disease agent and/or an antithyroid agent to a feline in conjunction with feeding the feline a composition to prevent a cardiovascular disease, (d) administering an anti-cardiovascular disease agent and/or an antithyroid agent to a feline with hyperthyroidism in conjunction with feeding the feline a composition to treat a cardiovascular disease, (e) preventing cardiovascular disease in a feline with hyperthyroidism by feeding the feline a composition of the invention, (f) treating a cardiovascular disease in a hyperthyroid feline by feeding the feline a composition of the invention, (g) preventing a cardiovascular disease in a hyperthyroid feline by administering to the feline an anti-cardiovascular disease agent and/or antithyroid agent in conjunction with feeding the feline a composition of the invention, (h) treating cardiovascular disease in a hyperthyroid feline by administering to the feline an anti-hyperthyroidism agent and/or antithyroid agent in conjunction with feeding the feline a composition of the invention, (i) using a kit to prevent a cardiovascular disease in a feline with hyperthyroidism, and j) using a kit to treat a cardiovascular disease in a feline with hyperthyroidism. The communication means comprise, for example, a document, digital storage media, optical storage media, audio presentation, or visual display containing the information or instructions. Preferably, the communication means is a displayed web site or a brochure, product label, package insert, advertisement, or visual display containing such information or instructions. Useful information or instructions include, for example, (1) information and instructions how to use a composition, method, or kit of the invention and (2) contact information for animal caregivers if they have a question about the invention and its uses.

In some embodiments, the invention provides a means for communicating information about or instructions for one or more of (a) preventing and/or treating a cardiovascular disease in a feline with hyperthyroidism by feeding the feline a composition that comprises from about 0.07 to less than about 1 mg/kg iodine and/or from about 0.1 to about 1.3 mg/kg selenium; (b) preventing and/or treating a cardiovascular disease in a hyperthyroid feline by administering to the feline an anti-cardiovascular disease agent and/or an antithyroid agent in conjunction with feeding the feline a composition that comprises from about 0.07 to less than about 1 mg/kg iodine and/or from about 0.1 to about 1.3 mg/kg selenium; and (c) using a kit that comprises a composition comprising from about 0.07 to less than about 1 mg/kg iodine and/or from about 0.1 to about 1.3 mg/kg selenium, or two or more ingredients that when combined together and, optionally, with additional ingredients, yield a composition that comprises from about 0.07 to less than about 1 mg/kg iodine and/or from about 0.1 to about 1 mg/kg selenium. The means comprise a document, digital storage media, audio presentation, or visual display containing the information or instructions.

Unless defined otherwise, all technical and scientific terms used herein have the same meanings as commonly understood by one of ordinary skill in the art to which this invention belongs.

As used herein, unless specifically stated otherwise, ranges are a shorthand for describing each and every value within a range, including endpoints. All references cited in the present disclosure are hereby incorporated by reference in their entirety. However, where there is a conflict between a definition in the present disclosure and that of a cited reference, the present disclosure controls.

Unless otherwise stated, all percentages provided herein are weight percentages on a dry matter basis, e.g., 31% protein means 31% protein on a dry matter basis. Also, the concentrations of ingredients are given, e.g., 1 mg/kg iodine means 1 mg/kg iodine on a dry matter basis. The term "dry matter basis" means that an ingredient's concentration in a composition is measured after removing the moisture from the composition.

The singular forms "a", "an", and "the" include plural reference unless the context clearly indicates otherwise. The terms "comprise", "comprises", and "comprising" are to be interpreted inclusively rather than exclusively.

As used herein, "an amount effective", "an effective amount", and like terms refer to that amount of a compound, material or composition as described herein that may be effective to achieve a particular biological result. Such effective activity may be achieved, for example, by administration of compositions of the present invention to an animal. An effective amount may be based on several factors, including an animal's ideal weight, the metabolizable energy of the composition, and frequency of feeding the animal compositions of the present invention, e.g., once, twice, or three times daily, and other compositions fed to the animal.

In the specification, there have been disclosed typical preferred embodiments of the invention and, although specific terms are employed, they are used in a generic and descriptive sense only and not for purposes of limitation, the scope of the invention being set forth in the clauses. Obviously many modifications and variations of the invention are possible in light of the above teachings. It is understood that within the scope of the appended clauses the invention may be practiced otherwise than as specifically described.

### EXAMPLES

The invention can be further illustrated by the following example, although it will be understood that the example is included merely for purposes of illustration and is not intended to limit the scope of the invention unless otherwise specifically indicated.

### EXAMPLE 1

Example 1 illustrates the effect of the compositions on preventing and/or treating cardiovascular diseases concurrently with treating hyperthyroidism in hyperthyroid cats.

Food A is formulated as a dry cat food containing 0.21 mg/kg iodine and 0.74 mg/kg selenium and meeting AAFCO's minimum nutrient level requirements for maintenance for cats. It contains soybean meal, corn gluten meal as protein ingredients. Food A contains approximately 97% vegetable and approximately 34% total protein.

Food B is formulated as a dry cat food containing 0.38 mg/kg iodine and 0.67 mg/kg selenium and meeting AAFCO's minimum nutrient level requirements for maintenance for cats. It contains soybean meal, corn gluten meal, poultry meal, and pork meat protein isolate as protein ingredients. Food B contains approximately 76% vegetable and approximately 34% total protein.

Food C is formulated as a dry cat food containing 0.25 mg/kg iodine and 0.95 mg/kg selenium, meeting AAFCO's minimum nutrient level requirements for maintenance for cats. It contains corn gluten meal, and pork meat protein isolate as protein ingredients. Food C contains approximately 85% vegetable and approximately 34% total protein.

Food D is formulated as a wet cat food containing 0.21 mg/kg iodine and 1.2 mg/kg selenium, meeting AAFCO's minimum nutrient level requirements for maintenance for cats. It contains soybean meal, pork lungs, and pork liver as protein ingredients. Food D contains approximately 32% vegetable and approximately 34% total protein.

Twenty two geriatric cats (average age is 13.9 years, range is 11.5 - 17.2 yrs) are identified as being hyperthyroid based on thyroid hormone profiles, thyroid scans, and T₃ suppression tests. Of the twenty two cats, 64% have elevated thyroid levels at baseline, the average TT₄ is 73.4 nmol/L, the TT₄ range is 30.9 - 145.4 (normal range = 10 - 55).

All twenty two cats have echocardiographic measurements at baseline and six months later following dietary treatment. Additionally, eighteen cats have baseline electrocardiograms. Seventeen cats are assigned to one of foods A, B, or C. The foods are fed to the cats for 24 weeks. Although all foods are formulated to contain around 0.25 mg/kg iodine, analytical results revealed that food B was highly variable in iodine ranging from about 0.27 to about 0.60 mg/kg iodine. The remaining five cats, intended to be fed wet food D, refused consumption. Thus, for this study, those cats are fed food C for 3 weeks, and then switched to a control senior diet food for the remainder of the study.

The electrocardiograms are evaluated for abnormalities in cardiac rhythm or conduction pattern and assessed for patterns of chamber enlargement. Eleven cats display seventeen ECG abnormalities. Of these eleven cats, eight (73%) have elevated thyroid levels at baseline. The results of the electrocardiographic findings and comparison to previous studies are shown in Table 1 below.

**Table 1: Results from Electrocardiography Measurements**

| **Parameter** | **Example 1 (18 cats)** | **Peterson et al. Study (131 cats)¹** | **Fox et al. Study (119 cats)²** |
|---|---|---|---|
| Sinus tachycardia | 8/18 (44%) | 66% | 34% |
| Left anterior fascicular branch (LAFB) | 3/18 (17%) | 6% | 8% |
| Prolonged QRS duration | 2/18 (11%) | 18% | 39% |
| Increased R wave amplitude in lead II (>0.9 mV) | 0/18 (0%) | 29% | 8% |

| | | | |
|---|---|---|---|
| ¹Peterson et al., Journal of the American Veterinary Medical Association 183:103-110 (1983) ² Fox et al., Journal of the American Animal Hospital Association 35:27-31 (1999) | | | |

Similar to previous studies, the most frequently identified abnormalities are evident in the left ventricular posterior wall (See Table 2 below). One of the five cats randomized to food A, two of the five cats randomized to food B, two of the seven cats randomized to food C, and two of the five cats randomized to food D displayed echocardiographic abnormalities. Of these seven cats, four have elevated TT₄ and three have normal TT₄ at baseline. With the exception of left atrial enlargement (LAE), the prevalence of hypertrophy for interventricular septum (IVS), left ventricular posterior wall (LVPW), and hyperdynamic left ventriculum (LVH) is similar to the ones observed by Moise et al., American Journal of Veterinary Research 47:1487-94 (1986). The LAE in the cats in Example 1 is much less prevalent (14 versus 55%) than the LAE observed by Moise et al. This may be attributed to earlier detection and/or absence of clinical signs of hyperthyroidism in the cats of Example 1.

**Table 2: Results from Echocardiography Measurements**

| **Parameter** | **Example 1 (22 cats)** | **Comments** | **Moise et al. (20 cats)** | **Criteria** |
|---|---|---|---|---|
| LAE | 3/22 | All 3 cats have elevated TT₄ | 11/20 | LA > or equal to 1.5cm |
| | (14%) | | (55%) | |
| IVS | 2/22 | Both cats have normal TT₄ | 2/20 | IVSd > or equal to 0.6cm |
| hypertrophy | (9%) | | (10%) | |
| LVPW | 6/22 | Four cats have elevated TT₄ | 6/20 | LVPWd > or equal to 0.6cm |
| hypertrophy | (27%) | | (30%) | |
| LVH | 0/22 | | 0/20 | LVDd > or equal to 1.7cm |
| | (0%) | | (0%) | |

Table 3 presents the results from the LVPW measurements for the six cats identified at baseline as having cardiac hypertrophy based on LVPW measurement (e.g., LVPW at diastole > 0.6 cm). From the results in Table 3, it appears that there is no improvement due to feeding foods A, B, C, or D. When, however, the mean changes in echocardiographic measurements are evaluated as affected by a specific food regimen (Table 4) or regressed on changes in TT₄, both a statistical significance and significant correlations **are observed for a number of cardiac measures (Table 5).**

**Table 3: Echocardiographic Changes in Hyperthyroid Cats Foods A, B, C, and D**

| **Food** | **Baseline TT₄** | **TT₄ at week 24** | **Pre-Food LVPW at Diastole** | **Post-Food LVPW at Diastole** |
|---|---|---|---|---|
| A | 131 | 37 | 0.67 | 0.65 |
| B (cat 1) | 130 | 72 | 0.67 | 0.70 |
| B (cat 2) | 81 | 70 | 0.73 | 0.80 |
| C | 79 | | 0.67 | |
| D (cat 1) | 55 | 43 | 0.77 | 0.80 |
| D (cat 2) | 37 | 42 | 0.60 | 0.59 |

| | | | | |
|---|---|---|---|---|
| TT₄ = Total T₄ | | | | |

**Table 4: Ultrasound Changes in Hyperthyroid Cats Foods A, B, C, and D**

| **Food** | **Iodine (ppm (DMB)** | **Baseline TT₄ (nmol/L)** | **TT₄ at week 24 (nmol/L)** | **TT₄ Change (nmol/L)** | **IVS Change at Systole** | **LVED Change at Diastole** | **LVPW Change at Diastole** |
|---|---|---|---|---|---|---|---|
| A | 0.21 (0.16-0.31) | 75.8 | 48 | -27.8 | 0.218 | 0.068 | -0.022 |
| B | 0.38 (0.27-0.60) | 76.4 | 63.2 | -13.2 | 0.433 | -0.078 | 0.115 |
| C | 0.25 (0.15-0.34) | 70 | 30 | -40.0 | 0.199 | 0.116 | -0.024 |
| D* | 0.21 (0.14-0.27) | 76.4 | 51.6 | -24.8 | 0.180 | 0.018 | -0.002 |

| | | | | | | | |
|---|---|---|---|---|---|---|---|
| *Cats are fed Food D for three weeks, and then switched to a senior control diet containing 2.5 ppm iodine; LVED = left ventricular end dimension | | | | | | | |

**Table 5: Significant Correlations between Ultrasound and Thyroid Hormone Profiles (r and P-value)**

| **Parameters** | **TSH at week 24** | **TSH Change** | **TT₃ Change** | **TT₄ at week 24** | **TT₄ Change** |
|---|---|---|---|---|---|
| IVS Change at Diastole | -0.661 (0.007) | -0.529 (0.043) | | 0.513 (0.025) | |
| IVS Change at Systole | -0.630 (0.012) | -0.539 (0.038) | | | |
| LVED Change at Systole | 0.558 (0.031) | | -0.520 (0.047) | -0.577 (0.010) | |
| LVPW Change at Diastole | | | | | 0.528 (0.043) |
| LVPW Change at Systole | | | 0.632 (0.011) | | 0.588 (0.021) |
| FS Change | | | | 0.564 (0.012) | |

| | | | | | |
|---|---|---|---|---|---|
| TSH = thyroid stimulating hormone; TT₃ = total T₃; FS = fractional shortening | | | | | |

There is a significant reduction in TT₄ in the cats fed foods A, B, and D. In addition, improvements in ultrasound measures (IVSs, LVEDd and LVPWd) are observed. There is no improvement in the ultrasound measurements for cats fed B, however, and this agrees with the lack of significant change in TT₄. With the exception of IVSs, the greatest improvement in cardiac measurements is observed for cats fed foods C>A>D>B, which correlates with the magnitude of reduction in TT₄. Concentric hypertrophy is defined by an increased interventricular septal or left ventricular free wall measurement. As a result of these structural increases, left ventricular chamber volume (e.g., left ventricular end-diastolic dimension LVEDd) is often smaller than normal. Thus, a decrease in IVSs and LVPWd and an increase in LVEDd are desired with successful treatment or resolution of hyperthyroid disease. In cats fed food D, a significant reduction in TT₄ is observed during the three weeks during which those cats are fed wet food D. This reduction in TT₄ is probably attributed to the unintentional dietary restriction or food refusal that occurred in these cats when cats are fed food D (all five cats lost bodyweight over that three week period) as body weight loss or severe food restriction have been shown to depress thyroid hormone production.

The apparent discrepancy between the data shown in Table 3 and the treatment mean data and correlation analyses shown in Tables 4 and 5 can probably be attributed to a number of factors including small number of animals, limited number of cats that present with "clinical" evidence of cardiac hypertrophy, insufficient study duration to demonstrate improvement in the cats with more advanced cardiac derangement, concurrent or confounding effect of other diseases such as renal insufficiency, which can affect echocardiographic changes. In addition, it is also possible that cats with more advanced stages of concentric hypertrophy and/or hyperthyroid disease will not resolve or improve with time. Cats fed foods A and C remained unchanged. This finding observation correlates with other studies that have observed that not all cardiac conditions resolve or improve following successful treatment of hyperthyroidism. The statistical changes in ultrasound measurements that are observed in Example 1 correlated very well with the thyroid profile changes and reductions in liver enzymes (alanine aminotransferase (ALT) and alkaline phosphatase (ALP)). In addition, the treatment rankings (e.g., food C>A>B) are consistent across all parameters measured. Feeding food C resulted in the greatest magnitude of reductions: 55% decrease in thyroid hormone profiles, 45% reduction in ALT, and 40% reduction in ALP. As all of these parameters have been associated with cats with hyperthyroidism, thus a reduction in these parameters would be expected with successful dietary treatment.

While particular embodiments of the present invention have been shown and described herein, it will be apparent to those skilled in the art that changes and modifications may be made without departing from the broader aspects of invention.

In particular, the invention as described herein provides the following embdoiments:
1. A method for preventing and/or treating a cardiovascular disease in a feline with hyperthyroidism comprising feeding the feline a composition that comprises from about 0.07 to less than about 1 mg/kg iodine on a dry matter basis.
2. The method of clause 1 wherein the composition comprises from about 0.1 to less than about 1 mg/kg iodine.
3. The method of clause 1 wherein the composition comprises from about 0.1 to about 0.5 mg/kg iodine.
4. The method of clause 1 wherein the composition further comprises from about 0.1 to about 1.3 mg/kg selenium.
5. The method of clause 1 wherein the composition further comprises from about 0.1 to about 1 mg/kg selenium.
6. The method of clause 1 wherein the composition further comprises from about 0.1 to about 0.8 mg/kg selenium.
7. The method of clause 1 wherein the composition further comprises from about 31 to about 35% protein wherein the protein comprises at least about 75% vegetable protein.
8. The method of any of clauses 1-7 wherein the method further comprises administering to the feline an antithyroid agent in conjunction with feeding the feline the composition.
9. The method of clause 8 wherein a therapeutically-effective amount of the antithyroid agent is administered to the feline.
10. The method of any of clauses 1-9 wherein the method further comprises administering to the feline an anti-cardiovascular disease agent in conjunction with feeding the feline the composition.
11. The method of clause 10 wherein a therapeutically-effective amount of the anti-cardiovascular disease agent is administered to the feline.
12. The method of clause 1 wherein the cardiovascular disease comprises cardiac hypertrophy.
13. The method of clause 1 wherein the cardiovascular disease comprises hypertrophic cardiomyopathy.
14. The method of clause 1 wherein the feline is a cat.
15. The method of clause 1 wherein the composition is a food composition.
16. A method for preventing and/or treating a cardiovascular disease in a feline with hyperthyroidism comprising feeding the feline a composition that comprises from about 0.1 to about 1.3 mg/kg selenium on a dry matter basis.
17. The method of clause 16 wherein the composition further comprises from about 0.1 to about 1 mg/kg selenium.
18. The method of clause 16 wherein the composition further comprises from about 0.1 to about 0.8 mg/kg selenium.
19. The method of clause 16 wherein the composition further comprises from about 0.15 to about 0.65 mg/kg selenium.
20. The method of any of clauses 16-19 wherein the composition further comprises from about 31 to about 35% protein wherein the protein comprises at least about 75% vegetable protein.
21. The method of clauses 16-19 wherein the method further comprises administering to the feline an antithyroid agent in conjunction with feeding the feline the composition.
22. The method of clause 20 wherein a therapeutically-effective amount of the antithyroid agent is administered to the feline.
23. The method of any of clauses 16-22 wherein the method further comprises administering to the feline an anti-cardiovascular disease agent in conjunction with feeding the feline the composition.
24. The method of clause 23 wherein a therapeutically-effective amount of the anti-cardiovascular disease agent is administered to the feline.
25. The method of clause 16 wherein the cardiovascular disease comprises cardiac hypertrophy.
26. The method of clause 16 wherein the cardiovascular disease comprises hypertrophic cardiomyopathy.
27. The method of clause 16 wherein the feline is a cat.
28. The method of clause 16 wherein the composition is a food composition.
29. A composition for preventing and/or treating a cardiovascular disease in a feline with hyperthyroidism comprising from about 0.07 to less than about 1 mg/kg iodine and/or from about 0.1 to about 1.3 mg/kg selenium on a dry matter basis.
30. The composition of clause 29 wherein the composition comprises from about 0.1 to less than about 1 mg/kg iodine.
31. The composition of clause 29 wherein the composition comprises from about 0.1 to about 0.5 mg/kg iodine.
32. The composition of clause 29 wherein the composition comprises from about 0.1 to about 0.8 mg/kg selenium.
33. The composition of clause 29 wherein the composition further comprises from about 31 to about 35% protein wherein the protein comprises at least about 75% vegetable protein.
34. The composition of any of clauses 29-33 wherein the cardiovascular disease comprises cardiac hypertrophy.
35. The composition of any of clauses 29-33 wherein the cardiovascular disease comprises hypertrophic cardiomyopathy.
36. The composition of any of clauses 29-33 wherein the feline is a cat.
37. The composition of any of clauses 29-33 wherein the composition is a food composition.
38. A kit for preventing and/or treating a cardiovascular disease in a feline with hyperthyroidism comprising in separate containers in a single package or in separate containers in a virtual package, as appropriate:
   a composition that comprises from about 0.07 to less than about 1 mg/kg iodine on a dry matter basis and/or from about 0.1 to about 1.3 mg/kg selenium on a dry matter basis, or two or more ingredients that, when combined together and, optionally, with additional ingredients that are or are not a part of the kit, yield a composition that comprises from about 0.07 to less than about 1 mg/kg iodine on a dry matter basis and/or from about 0.1 to about 1.3 mg/kg selenium on a dry matter basis, and one or more of:
      (a) an anti-cardiovascular disease agent;
      (b) an antithyroid agent;
      (c) instructions for preparing the composition;
      (d) instructions for feeding the composition to a feline with hyperthyroidism;
      (e) instructions for preventing and/or treating a cardiovascular disease in a feline with hyperthyroidism by feeding the feline the composition; and
      (f) instructions for preventing and/or treating a cardiovascular disease in a feline with hyperthyroidism by administering to the feline an anti-cardiovascular disease agent and/or an antithyroid agent in conjunction with feeding the feline the composition.
39. A means for communicating information about or instructions for one or more of:
   (a) preventing and/or treating a cardiovascular disease in a feline with hyperthyroidism by feeding the feline a composition that from about 0.07 to less than about 1 mg/kg iodine on a dry matter basis and/or from about 0.1 to about 1.3 mg/kg selenium on a dry matter basis;
   (b) preventing and/or treating a cardiovascular disease in a feline with hyperthyroidism by administering to the feline an anti-cardiovascular disease agent and/or an antithyroid agent in conjunction with feeding the feline a composition that comprises from about 0.07 to less than about 1 mg/kg iodine on a dry matter basis and/or from about 0.1 to about 1.3 mg/kg selenium on a dry matter basis, and
   (c) using a kit of clause 38,
   the means comprising a document, digital storage media, audio presentation, or visual display containing the information or instructions.

## Claims

1. Iodine and vegetable protein for use in preventing and/or treating a cardiovascular disease in a feline with hyperthyroidism,
wherein the use comprises administering iodine and vegetable protein in the form of a food composition,
wherein the composition comprises from about 0.07 to less than about 1 mg/kg iodine on a dry matter basis, and from about 31% to about 35% protein,
and wherein the protein comprises at least about 75% vegetable protein.

2. The composition for use according to claim 1 wherein the composition comprises from about 0.1 to less than about 1 mg/kg iodine on a dry matter basis.

3. The composition for use according to claim 1 or claim 2 wherein the composition comprises from about 0.1 to less than about 1 mg/kg iodine, or from about 0.1 to about 0.5 mg/kg iodine; and/or further comprises from about 0.1 to about 1.3 mg/kg selenium, or from about 0.1 to about 1 mg/kg selenium, or from about 0.1 to about 0.8 mg/kg selenium.

4. The composition for use according to any of claims 1 to 3, wherein the composition comprises from about 0.1 to less than about 1 mg/kg iodine, or from about 0.1 to about 0.5 mg/kg iodine; and/or further comprises from about 0.1 to less than about 1 mg/kg selenium, or from about 0.1 to about 0.8 mg/kg selenium.

5. Selenium and vegetable protein for use in preventing and/or treating a cardiovascular disease in a feline with hyperthyroidism,
wherein the use comprises administering selenium and vegetable protein in the form of a food composition,
wherein the composition comprises from about 0.1 to about 1.3 mg/kg selenium on a dry matter basis, and from about 31% to about 35% protein,
and wherein the protein comprises at least about 75% vegetable protein.

6. The composition for use according to claim 5 wherein the composition comprises from about 0.1 to about 1 mg/kg selenium on a dry matter basis.

7. The composition for use according to claim 5 or claim 6 wherein the composition further comprises from about 0.1 to about 1 mg/kg selenium or from about 0.1 to about 0.8 mg/kg selenium, or from about 0.15 to about 0.65 mg/kg selenium.

8. The composition for use according to any preceding claim in conjunction with an antithyroid agent as a combined preparation for use in preventing and/or treating cardiovascular disease in a feline with hyperthyroidism.

9. The composition for use according to claim 8 wherein the combined preparation further comprises an anti-cardiovascular disease agent.

10. Iodine, selenium and vegetable protein for use in preventing and/or treating a cardiovascular disease in a feline with hyperthyroidism,
wherein the use comprises administering iodine, selenium and vegetable protein in the form of a food composition,
wherein the composition comprises from about 0.07 to less than about 1 mg/kg iodine and from about 0.1 to about 1.3 mg/kg selenium on a dry matter basis, and from about 31% to about 35% protein,
wherein the protein comprises at least about 75% vegetable protein.

11. The composition for use according to claim 10, wherein the composition comprises from about 0.1 to less than about 1 mg/kg iodine and from about 0.1 to less than about 1 mg/kg selenium on a dry matter basis.

12. The composition for use according to claim 10 or claim 11 wherein the composition comprises from about 0.1 to less than about 1 mg/kg iodine, or from about 0.1 to about 0.5 mg/kg iodine, or from about 0.1 to about 0.8 mg/kg selenium.

13. The composition for use according to any preceding claim wherein the cardiovascular disease comprises cardiac hypertrophy.

14. The composition for use according to any of claims 1 to 12 wherein the cardiovascular disease comprises hypertrophic cardiomyopathy.

15. The composition for use according to any preceding claim wherein the feline is a cat.
